# EUROPÄISCHE PATENTSCHRIFT

(11) **EP 2 991 610 B1**
(45) Veröffentlichungstag und Bekanntmachung des Hinweises auf die Patenterteilung: **18.12.2019**
(21) Anmeldenummer: 14720971.2
(22) Anmeldetag: 30.04.2014
(51) Int. Cl.: A61K 6/027, C03C 4/00, C03C 10/00, C03B 32/02, A61C 13/00

(54) **VERFAHREN ZUR HERSTELLUNG EINES ROHLINGS SOWIE ROHLING**
PROCESS FOR PRODUCING A BLANK, AND A BLANK
PROCÉDÉ DE PRODUCTION D'UNE ÉBAUCHE ET ÉBAUCHE CORRESPONDANTE

(30) Priorität: 03.05.2013 DE 102013104561
(43) Veröffentlichungstag der Anmeldung: 09.03.2016
(60) Teilanmeldung: 16189583.4
(73) Patentinhaber: Dentsply Sirona Inc., York, PA 17401 (US)
(72) Erfinder: FECHER, Stefan, 63867 Johannesberg (DE); HÖRHOLD, Heiner, 63654 Büdingen (DE); SCHUSSER, Udo, 63755 Alzenau (DE); VOLLMANN, Markus, 63571 Gelnhausen (DE); KUTZNER, Martin, 63543 Neuberg (DE)
(74) Vertreter: Dietz, Mirko
(86) Internationale Anmeldenummer: PCT/EP2014/058920
(87) Internationale Veröffentlichungsnummer: WO 2014/177659

(56) Entgegenhaltungen:
- EP-A1- 2 377 831
- DE-A1-102010 056 037
- US-A1- 2008 120 994
- US-A1- 2012 309 607
- US-B1- 6 455 451
- OLIVEIRA DE A P N ET AL: "SINTERING AND CRYSTALLIZATION OF A GLASS POWDER IN THE LI2O-ZRO2- SIO2 SYSTEM", JOURNAL OF THE AMERICAN CERAMIC SOCIETY, BLACKWELL PUBLISHING, MALDEN, MA, US, vol. 81, no. 3, 1 March 1998 (1998-03-01), pages 777-780, XP000738310, ISSN: 0002-7820
- BOROM ET AL: "Strength and Microstructure in Lithium Disilicate Glass-Ceramics", JOURNAL OF THE AMERICAN CERAMIC SOCIETY, vol. 58, no. 9,10, 1975, pages 385-391,
- Hollemann, Wiberg: "Lehrbuch der Anorganischen Chemie", 1995, de Gruyter, Berlin page 911,

## Beschreibung

Die Erfindung bezieht sich auf ein Verfahren zur Herstellung eines dentalen Formteils, wie Inlay, Onlay, Krone oder Brücke.

Der WO 2012/080513 A1 ist ein Verfahren zur Herstellung dentaler Formteile aus porösem Glas zu entnehmen, das kristalline Anteile nicht aufweist. Die Dichte des Rohlings liegt zwischen 50 % und 95 % der theoretischen Dichte eines dichtgesinterten Rohlings. Aus einem diesbezüglichen Rohling werden monolithische dentale Formteile wie Kronen, Teilkronen, Brücken, In- oder Onlays mittels Fräsen hergestellt, wobei eine trockene Bearbeitung erfolgt.

Der WO 2011076422 A1 oder der WO 2012/059143 A1 sind Lithiumdisilikat-Glaskeramiken zu entnehmen, die zur Herstellung von dentalen Formteilen verwendet werden. Die entsprechenden Glaskeramiken sollen sich durch gute mechanische und optische Eigenschaften auszeichnen.

Aus der WO 2013/053865 A2 ist ein Rohling aus einer Lithiumsilikat-Glaskeramik bekannt, aus dem dentale Formteile hergestellt werden. Die Keramik enthält zwingend ein dreiwertiges Metalloxid aus der Gruppe Y₂O₃, La₂O₃, Yb₂O₃, Bi₂O₃ und Mischungen dieser. Des Weiteren ist die Glaskeramik im Wesentlichen frei von K₂O und Na₂O.

Um Zahnersatz auf der Basis von Lithiumdisilikat-Glaskeramik herzustellen, ist es auch bekannt, zylinderförmige Pellets herzustellen und diese sodann in einer Muffel zu verpressen (EP 1 484 031 B1). EP 2 377 831 A1 beschreibt ebenfalls Lithiumglaskeramiken mit Gehalt an Zr02 zur Verpressung in Muffeln.

Die US 6 455 451 B1 beschreibt einen Rohling zur Herstellung eines dentalen Formteils, wobei der Rohling aus Glaskeramik einen Anteil von 35-60 Vol.-% an Lithiumsilikatkristallen enthält.

Aus der US 2012/0309607 A1 ist ein Rohling zur Herstellung eines dentalen Formteils bekannt, wobei der Rohling aus Glaskeramik einen Anteil von mehr als 10 Vol.-% an Lithiumsilikatklistallen enthält.

Ein Rohling zur Herstellung eines dentalen Formteils wird in der US 2008/120 994 A1 beschrieben. Der aus Glaskeramik bestehende Rohling weist einen Anteil von 60-80 Vol.-% an Lithiumsilikatkristallen auf.

Ein Rohling zur Herstellung eines dentalen Formteils mit einer Dichte zwischen 50 und 65 % der theoretischen Dichte ist aus der DE 10 2010 056 037 A1 bekannt. Glaskeramiken auf Basis eines Li2O-ZrO2-SiO2 Systems sind auch aus J. Am. Ceram. Soc. 81(3),777-780 (1998) bekannt.

Der vorliegenden Erfindung liegt die Aufgabe zu Grunde, ein Verfahren zur Herstellung dentaler Formteile mit guten mechanischen Eigenschaften bereitzustellen. Die Erfindung zeichnet sich insbesondere auch aus durch ein Verfahren zur Herstellung eines dentalen Formteils, wie Inlay, Onlay, Krone oder Brücke, umfassend die Verfahrensschritte
- Herstellen einer Schmelze einer Zusammensetzung in Gew.-%:

| | |
|---|---|
| SiO2 | 46,0 - 72,0 |
| Li2O | 10,0 - 25,0 |
| ZrO2 | 6,5 - 14,0 |
| P2O5 | 1,0 - 10,0 |
| Al2O3 | 0,1 - 8,0 |
| K2O | 0,1 - 5,0 |
| CeO2 | 0,1 - 4,0 |
| B2O3 | 0,0 - 4,0 |
| Na2O | 0,0 - 4,0 |
| Tb4O7 | 0,0 - 2,5 |

sowie 0,0 bis 4,0 von zumindest einem Additiv,
- Herstellen einer Glasfritte durch Verdüsen der Schmelze und Abschrecken in einem Medium,
- gegebenenfalls Erzeugen von Glaspulverpartikeln aus der Glasfritte mit einer Korngrößenverteilung d₉₀ ≤ 80 µm,
- Auskristallisieren von Lithiumsilikatkristallen mit einem Volumenanteil zwischen 10 % und 90 % durch eine erste Wärmebehandlung von entweder der Glasfritte oder den Glaspulverpartikeln in einem ersten Temperaturbereich bei einer Temperatur T₁ mit 500 °C ≤ T₁ ≤ 750 °C über eine Zeit t₁ mit 5 min ≤ t₁ ≤ 120 min,
- wobei dann, wenn die Glasfritte einer Wärmebehandlung unterzogen worden ist, Herstellen von Glaskeramikpartikeln mit einer Korngrößenverteilung d₉₀ ≤ 80 µm aus der wärmebehandelten Glasfritte erfolgt,
- Pressen der Glaskeramikpulverpartikel zu einem Rohling mit einer Dichte zwischen 30 % und 60 % der theoretischen Dichte des Rohlingmaterials von etwa 2,6 g/cm³,
- Bearbeiten des Rohlings durch Fräsen zur Herstellung eines dem dentalen Formteil entsprechenden Vorformteils unter Berücksichtigung des Schwundverhaltens des Rohlings und
- Durchsintem des Vorformteils bei einer Temperatur T₂ mit 800 °C ≤ T₂ ≤ 1050 °C über eine Zeit t₂ mit 5 min ≤ t₂ ≤ 60 min.

Erfindungsgemäß besteht die Möglichkeit, dass entweder die Glasfritte, ohne dass diese zuvor gemahlen wird, um Glaspulverpartikel zu erhalten, einer Wärmebehandlung zur Bildung von Lithiumsilikatkristallen unterzogen wird, oder aber die Fritte zunächst gemahlen wird, also Glaspulverpartikel erzeugt werden, um sodann die Wärmebehandlung durchzuführen, so dass sodann Glaskeramikpulverpartikel vorliegen. Das Merkmal "Pressen der Glaskeramikpulverpartikel zu einem Rohling" umfasst folglich die nach den Verfahrensalternativen hergestellten Glaskeramikpulverpartikel.

Dabei besteht die Möglichkeit, die erste Wärmebehandlung zweistufig innerhalb des ersten Temperaturbereichs durchzuführen.

Insbesondere weist die Schmelze eine Zusammensetzung in Gew.-% auf:

| | |
|---|---|
| SiO2 | 49,0 - 69,0 |
| Li2O | 11,5 - 24,0 |
| ZrO2 | 7,0 - 13,5 |
| P2O5 | 1,5 - 9,0 |
| Al2O3 | 0,2 - 7,5 |
| K2O | 0,2 - 4,5 |
| CeO2 | 0,2 - 3,5 |
| B2O3 | 0,0 - 3,5 |
| Na2O | 0,0 - 3,5 |
| Tb4O7 | 0,0 - 2,0 |

sowie 0,0 bis 4,0 von zumindest einem Additiv.

Bevorzugterweise besteht die Schmelze aus einer Zusammensetzung in Gew.-%:

| | |
|---|---|
| SiO2 | 52,0 - 66,0 |
| Li2O | 12,0 - 22,5 |
| ZrO2 | 7,5 - 13,0 |
| P2O5 | 2,0 - 8,5 |
| Al2O3 | 0,3 - 7,0 |
| K2O | 0,3 - 4,0 |
| CeO2 | 0,3 - 3,5 |
| B2O3 | 0,0 - 3,0 |
| Na2O | 0,0 - 3,0 |
| Tb4O7 | 0,0 - 2,0 |

sowie 0,0 bis 4,0 von zumindest einem Additiv.

Besonders hervorzuheben ist eine Zusammensetzung der Schmelze mit einer Zusammensetzung in Gew.-%:

| | |
|---|---|
| SiO2 | 55,0 - 63,0 |
| Li2O | 12,5 - 21,5 |
| ZrO2 | 8,0 - 12,0 |
| P2O5 | 2,5 - 8,0 |
| Al2O3 | 0,4 - 6,5 |
| K2O | 0,4 - 4,0 |
| CeO2 | 0,5 - 3,0 |
| B2O3 | 0,0 - 3,0 |
| Na2O | 0,0 - 3,0 |
| Tb4O7 | 0,0 - 2,0 |

sowie 0,0 bis 4,0 von zumindest einem Additiv.

Bevorzugterweise ist vorgesehen, dass die Schmelze eine Zusammensetzung in Gew.-% aufweist:

| | |
|---|---|
| SiO2 | 58,0 - 60,0 |
| Li2O | 13,5 - 20,5 |
| ZrO2 | 8,5 - 11,5 |
| P2O5 | 3,0 - 7,5 |
| Al2O3 | 0,5 - 6,0 |
| K2O | 0,5 - 3,5 |
| CeO2 | 0,5 - 2,5 |
| B2O3 | 0,0 - 3,0 |
| Na2O | 0,0 - 3,0 |
| Tb4O7 | 0,0 - 1,5 |

sowie 0,0 bis 4,0 von zumindest einem Additiv.

Das zumindest eine Additiv ist zumindest ein Additiv aus der Gruppe Farbpigment, Fluoreszenzmittel. Insbesondere ist vorgesehen, dass das Additiv zumindest ein Oxid aus der Gruppe BaO, CaO, MgO, MnO, Er2O3, Gd2O3, Pr6O11, Sm2O3, TiO2, V2O5, Y203 ist oder ein solches Oxid enthält.

Nach einem weiteren Vorschlag ist vorgesehen, dass der Rohling nach der ersten Wärmebehandlung und vor der mechanischen Bearbeitung getempert wird bei einer Temperatur T₃ mit 750 °C ≤ T₃ ≤ 900 °C über eine Zeit t₃ mit 5 min ≤ t₃ ≤ 30 min. Durch die diesbezüglichen Wärmebehandlungsschritte wird sichergestellt, dass das Glaspulver im erforderlichen Umfang zu Lithiumsilikatkristallen kristallisiert, gleichzeitig eine geringe Porengröße und damit dichte Kompackung ergibt, so dass eine problemlose mechanische Bearbeitung u. a. zur Erzielung filigraner Bereiche ermöglicht wird.

Die Kristallphase Lithiumsilikat schließt dabei Lithiummetasilikat und insbesondere Lithiumdisilikat ein.

Insbesondere ist vorgesehen, dass zur Herstellung eines Rohling in Scheibengeometrie die Glaskeramikpulverpartikel zunächst axial verpresst und sodann nach Einbringen in eine Umhüllende wie Beutel aus innenseitig beschichtetem Polyethylen isostatisch nachverdichtet werden, wobei das Nachverdichten insbesondere bei einem Druck pn mit 250 MPa ≤ pn ≤ 350 MPa über eine Zeit t₄ mit 5 sec ≤ t₄ ≤ 30 sec, insbesondere 5 sec ≤ t₄ ≤ 15 sec durchgeführt wird.

Zur Herstellung eines Rohlings in Quadergeometrie sieht die Erfindung vor, dass die Glaskeramikpulverpartikel sukzessiv und insbesondere kontinuierlich mit ansteigendem Druck über eine Zeit t₅ axial verpresst werden, wobei maximaler Druck p₅ beträgt 50 MPa ≤ p₅ ≤ 400 MPa, insbesondere 100 MPa ≤ p₅ ≤ 200 MPa. Der Zeitraum, innerhalb dem der Druckanstieg erfolgt, beträgt 10 sec ≤ t5 ≤ 20 sec.

Zur Herstellung eines Rohlings stabförmiger, insbesondere zylindrischer Geometrie, ist vorgesehen, dass das Glaskeramikpulver in eine schlauchförmige Pressform insbesondere aus Polyurethan eingebracht und sodann quasi isostatisch verpresst wird. Dabei sollten insbesondere nachstehende Presszeiten und Parameter berücksichtigt werden. Es wird zunächst ein langsamer Druckanstieg bevorzugt, um das eingefüllte Glaskeramikpulver gleichmäßig in der Form zu verteilen. Danach kann der Druck rasch auf seinen Maximalwert erhöht werden. Nach Erreichung des maximalen Drucks wird dieser für die Haltezeit konstant gehalten. Es folgt eine Phase der schnellen Druckentlastung, bei der der Druck bis auf 10% des Maximalwerts abgebaut wird. Der vollständige Abbau des Überdrucks erfolgt danach langsam, um Rissbildungen im Glaskeramikrohling zu vermeiden.

Zum mechanischen Bearbeiten, wobei eine trockene Bearbeitung möglich ist, ist insbesondere vorgesehen, dass zunächst eine Grobbearbeitung und sodann eine Feinbearbeitung erfolgt.

Bei der Grobbearbeitung sind bevorzugte Fräsparameter:

| | |
|---|---|
| Fräserdurchmesser: | 2 bis 5 mm, besonders 2 bis 3 mm |
| Vorschub: | 500 bis 4000 mm/min, besonders 2000 bis 3000 mm/min |
| Seitliche Zustellung ae: | 0,2 bis 3 mm, besonders 1 mm bis 2 mm |
| Tiefenzustellung ap: | 0,1 bis 2 mm, besonders 0,5 mm bis 1 mm |
| Fräserdrehzahl: | 10.000 bis 50.000 1/min, besonders 10.000 bis 20.000 1/min. |

Als Fräser sind Hartmetall-Fräser zu bevorzugen.

Bezüglich der Feinbearbeitung sollten nachstehende Fräsparameter beachtet werden:

| | |
|---|---|
| Fräserdurchmesser: | 0,3 bis 1,5 mm, besonders 0,5 bis 1,0 mm |
| Vorschub: | 300 bis 2000 mm/min, besonders 800 bis 1500 mm/min |
| Seitliche Zustellung ae: | 0,2 bis 0,6 mm, besonders 0,1 mm bis 0,2 mm |
| Tiefenzustellung ap: | 0,05 bis 0,3 mm, besonders 0,1 mm bis 0,15 mm |
| Fräserdrehzahl: | 20.000 bis 60.000 1/min, besonders 25.000 bis 35.000 1/min. |

Auch hierbei sind Hartmetall-Fräser zu bevorzugen.

Besonders gute Bearbeitungsergebnisse zeigen sich dann, wenn als Fräser ein Kugelradiusfräser aus Hartmetall verwendet wird, wobei sich der Kugelradiusfräser durch nachstehende Schneidenwinkel auszeichnen sollte:

| | |
|---|---|
| Span winkel: | 0° bis -13°, besonders -9° bis -11° |
| Freiwinkel: | 0° bis 15°, besonders 11° bis 13° |
| Keilwinkel: | ergibt sich 90° minus Freiwinkel minus Spanwinkel |

Ist es dem Grunde nach nicht erforderlich, dass vor dem Verpressen der Glaskeramikpartikel ein Bindemittel zugegeben wird, so wird die Erfindung nicht verlassen, wenn entsprechende Bindemittel wie z. B. Celluloseether mit einem Gewichtsanteil von bis zu 5 % zugegeben werden.

Insbesondere hat sich jedoch als Vorteil erwiesen, wenn der Rohling, also dessen Glaskeramikpartikel nach dem Verpressen in Kieselsäure oder Alkalisilikatlösung (Wasserglas) getaucht werden und nach dem Trocknen mechanisch bearbeitet werden. Hierdurch bilden sich zwischen den Glaspartikeln SiO₂-Brücken aus, durch die die Festigkeit gesteigert und somit die anschließende mechanische Bearbeitung, die ein CAD-/CAM-Bearbeiten einschließt, erleichtert wird. Beim Durchsintern der bearbeiteten Formteile diffundiert das freie SiO₂ in die Glaskeramik, wodurch eine Festigkeitserhöhung erzielbar ist.

Um die Glaskeramikpartikel zu verpressen, wird insbesondere ein Druck zwischen 50 MPa und 400 MPa, insbesondere zwischen 100 MPa und 200 MPa gewählt. Die Temperatur beim Vorsintern des kompaktierten Glaspulvers, also des Rohlings in Form des gepressten Glaskörpers, sollte im Bereich zwischen 500 °C und 950 °C, vorzugsweise zwischen 600 °C und 700 °C liegen.

Die Außengeometrie des gepressten Glaskeramikkörpers kann scheiben- bzw. plattenförmig oder stangen- wie zylinderförmig sein, wobei die Querschnittsgeometrie frei wählbar ist. Der Volumeninhalt der Rohlinge kann zwischen 1 cm³ und 160 cm³ liegen.

Nach dem mechanischen Bearbeiten der aus der kristallinen porösen Glaskeramik bestehenden Rohlinge, wobei bevorzugterweise eine Fräsbearbeitung ohne Kühlung erfolgt, werden die herausgearbeiteten dentalen Arbeiten in einem geeigneten Sinterofen unter Berücksichtigung eines geeigneten Temperatur-/Zeitzyklusses durchgesintert. Das Durchsintern kann dabei in einem Temperaturbereich zwischen 700 °C und 1100 °C, vorzugsweise im Bereich zwischen 850 °C und 950 °C durchgeführt werden. Die Gesamtzykluszeit beträgt weniger als 2 h, vorzugsweise weniger als 1 h. Aufgrund des kristallinen Anteils ist dabei nicht erforderlich, dass das Vorformteil gestützt wird. Vielmehr kann das Vorformteil z. B. auf einer Al₂O₃-Brennwatte im Sinterofen gelagert werden.

Als bevorzugter Temperatur-/Zeitzyklus ist anzugeben: Bereitschaftstemperatur 500 °C, Anstiegsrate 50°C/min bis 90°C/min auf 850 bis 900 °C, Haltezeit 1 bis 5 min, dann langsam abkühlen. Für die Abkühlung ist vorzugsweise die langsamste Kühlstufe zu wählen.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus den nachfolgenden Ausführungsbeispielen.

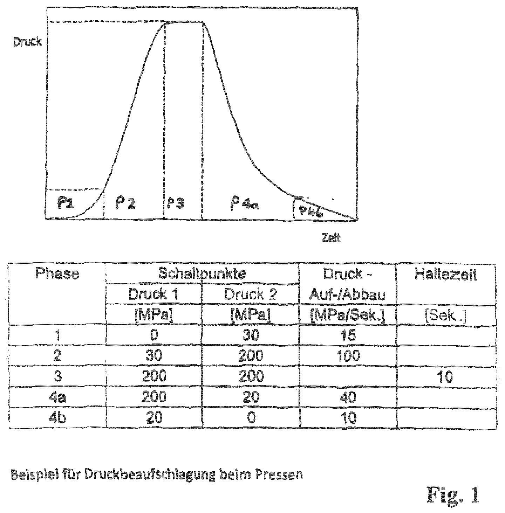

Die einzige Figur zeigt einen Verlauf des Drucks über die Zeit beim Verpressen eines Rohlings.

Um ein dentales Formteil herzustellen, wird erfindungsgemäß ein Rohling benutzt, der aus verpresstem Glaskeramikpulver besteht. Um das Glaskeramikpulver zur Verfügung zu stellen, wird zunächst ein Pulver aufgeschmolzen und aus der Schmelze eine Glasfritte hergestellt, die nachstehend bevorzugte Zusammensetzungen aufweisen kann:

| | |
|---|---|
| SiO2 | 49,0 - 69,0 |
| Li2O | 11,5 - 24,0 |
| ZrO2 | 7,0 - 13,5 |
| P2O5 | 1,5 - 9,0 |
| Al2O3 | 0,2 - 7,5 |
| K2O | 0,2 - 4,5 |
| CeO2 | 0,2 - 3,5 |
| B2O3 | 0,0 - 3,5 |
| Na2O | 0,0 - 3,5 |
| Tb4O7 | 0,0 - 2,0 |

sowie 0,0 bis 4,0 von zumindest einem Additiv.

Insbesondere ist vorgesehen, dass die Glasschmelze eine Zusammensetzung in Gew.-% aufweist:

| | |
|---|---|
| SiO2 | 49,0 - 69,0 |
| Li2O | 11,5 - 24,0 |
| ZrO2 | 7,0 - 13,5 |
| P2O5 | 1,5 - 9,0 |
| Al2O3 | 0,2 - 7,5 |
| K2O | 0,2 - 4,5 |
| CeO2 | 0,2 - 3,5 |
| B2O3 | 0,0 - 3,5 |
| Na2O | 0,0 - 3,5 |
| Tb4O7 | 0,0 - 2,0 |

sowie 0,0 bis 4,0 von zumindest einem Additiv.

Bevorzugterweise besteht die Galsschmelze aus einer Zusammensetzung in Gew.-%:

| | |
|---|---|
| SiO2 | 52,0 - 66,0 |
| Li2O | 12,0 - 22,5 |
| ZrO2 | 7,5 - 13,0 |
| P2O5 | 2,0 - 8,5 |
| Al2O3 | 0,3 - 7,0 |
| K2O | 0,3 - 4,0 |
| CeO2 | 0,3 - 3,5 |
| B2O3 | 0,0 - 3,0 |
| Na2O | 0,0 - 3,0 |
| Tb4O7 | 0,0 - 2,0 |

sowie 0,0 bis 4,0 von zumindest einem Additiv.

Besonders hervorzuheben ist eine Zusammensetzung der Glasschmelze mit einer Zusammensetzung in Gew.-%:

| | |
|---|---|
| SiO2 | 55,0 - 63,0 |
| Li2O | 12,5 - 21,5 |
| ZrO2 | 8,0 - 12,0 |
| P2O5 | 2,5 - 8,0 |
| Al2O3 | 0,4 - 6,5 |
| K2O | 0,4 - 4,0 |
| CeO2 | 0,5 - 3,0 |
| B2O3 | 0,0 - 3,0 |
| Na2O | 0,0 - 3,0 |
| Tb4O7 | 0,0 - 2,0 |

sowie 0,0 bis 4,0 von zumindest einem Additiv.

Bevorzugterweise ist vorgesehen, dass die Glasschmelze eine Zusammensetzung in Gew.-% aufweist:

| | |
|---|---|
| SiO2 | 58,0 - 60,0 |
| Li2O | 13,5 - 20,5 |
| ZrO2 | 8,5 - 11,5 |
| P2O5 | 3,0 - 7,5 |
| Al2O3 | 0,5 - 6,0 |
| K2O | 0,5 - 3,5 |
| CeO2 | 0,5 - 2,5 |
| B2O3 | 0,0 - 3,0 |
| Na2O | 0,0 - 3,0 |
| Tb4O7 | 0,0 - 1,5 |

sowie 0,0 bis 4,0 von zumindest einem Additiv.

Das zumindest eine Additiv ist zumindest ein Additiv aus der Gruppe Farbpigment, Fluoreszenzmittel. Insbesondere ist vorgesehen, dass das Additiv zumindest ein Oxid aus der Gruppe BaO, CaO, MgO, MnO, Er2O3, Gd2O3, Pr6O11, Sm2O3, TiO2, V2O5, Y203 ist oder ein solches Oxid enthält.

Das entsprechende Gemisch von Ausgangsstoffen, z. B. in Form von Oxiden und Carbonaten, wird sodann in einem geeigneten Tiegel aus feuerfestem Material oder Edelmetalllegierung bei einer Temperatur zwischen 1350 °C und 1600 °C über einen Zeitraum zwischen 1 h bis 10 h, insbesondere über einen Zeitraum von 4 h bis 7 h bei einer Temperatur von 1540 °C geschmolzen. Gleichzeitig oder anschließend erfolgt ein Homogenisieren, z. B. durch Rühren. Das so hergestellte flüssige Glas wird sodann einer vorzugsweise in Schwingung versetzten Düse zugeführt, die selbst auf eine Temperatur vorzugsweise im Bereich zwischen 1250 °C und 1450°C, insbesondere bei 1310 °C eingestellt ist. Die Düse kann einen Durchmesser zwischen 1 mm und 2 mm aufweisen. Die Schwingungsfrequenz der Düse kann zwischen 40 Hz und 60 Hz, insbesondere im Bereich von 50 Hz liegen. Das flüssige Glas wird sodann in einem geeigneten Medium wie Flüssigkeit, wie Wasser oder Hochtemperaturwatte abgeschreckt. Die so hergestellte abgeschreckte Glasfritte wird anschließend getrocknet. Sodann erfolgt ein Mahlen z. B. in einer Kugelmühle. Es erfolgt ein Sieben, wobei ein Sieb mit einer Maschenweite zwischen 50 µm und 500 µm benutzt werden kann. Nach Bedarf erfolgt ein weiteres Mahlen, z. B. durch eine Strahlenmühle (Jet mill) oder eine Attritionsmühle.

Aus dem so hergestellten Glas- bzw. Glaspartikelpulver werden insbesondere diejenigen ausgewählt, die einer Korngrößenverteilung d₉₀ ≤ 80 µm, insbesondere 10 µm ≤ d₅₀ ≤ 60 µm entsprechen. d₉₀ bzw. d₅₀ bedeutet, dass 90 % bzw. 50 % der vorhandenen Partikel einen Durchmesser besitzen, der kleiner als der angegebene Wert ist bzw. in dem Bereich liegen.

Damit der Rohling problemlos bearbeitet werden kann, ohne dass das aus dem Rohling hergestellte Formteil beim Durchsintern instabil ist, wird entweder die nach dem Schmelzen erhaltene Fritte oder das vor- bzw. endgemahlene Pulver einem Kristallisationsschritt unterzogen. Dabei wird in einem ersten Wärmebehandlungsschritt die Fritte bzw. das Pulver einer Temperatur T₁ zwischen 500 °C und 750 °C über eine Zeit t₁ zwischen 5 min und 120 min unterworfen. Der erste Wärmebehandlungsschritt kann auch zweistufig erfolgen, d. h. erster Wärmebehandlungsschritt 640 °C vorzugsweise 660 °C für 60 min und 750 °C für 40 min.

Bevorzugterweise schließt sich sodann eine weitere Wärmebehandlung in Form von Tempern an, wobei die zu wählende Temperatur T₃ zwischen 750 °C und 900 °C liegen sollte. Der Temperschritt wird über einen Zeitraum t₃ insbesondere zwischen 5 min und 30 min durchgeführt.

Sodann werden die Glaskeramikpartikel verpresst, wobei in Abhängigkeit von der herzustellenden Geometrie geeignete Pressverfahren, insbesondere ein axiales oder isostatisches Pressen oder Kombinationen dieser zur Anwendung gelangen. Das Verdichten erfolgt dabei in einem Umfang, dass die Dichte des Rohlings 30 % bis 60 % der theoretischen Dichte des Rohlingmaterials von etwa 2,64 g/cm³ entspricht. Insbesondere sollte der Rohling eine Dichte von in etwa 50 % der theoretischen Dichte aufweisen.

Zum Verpressen des Glaskeramikpulvers wird dieses vorzugsweise einem Druck zwischen 50 MPa und 400 MPA, insbesondere zwischen 100 MPa und 200 MPa ausgesetzt.

Die einzige Figur zeigt beispielhaft einen Verlauf des Drucks über die Zeit beim Verpressen eines Rohlings. In einer ersten Phase P1 wird der Druck ausgehend von 0 mit einem Druckaufbau von beispielsweise 15 MPa/sec auf einen Druck von beispielsweise 30 MPa erhöht. In einer zweiten Phase P2 wird der Druck von 30 MPa mit einem Druckaufbau von 100 MPa/sec auf einen Druck von ca. 200 MPa erhöht. In einer dritten Phase P3 wird der Druck auf einen Wert von ca. 200 MPa für eine Haltezeit von ca. 10 sec konstant gehalten. In einer vierten Phase erfolgt vorzugsweise ein zweistufiger Druckabbau, wobei in einer Phase P4a der Druck von ca. 200 MPa auf ca. 20 MPa mit einem Druckabbau von 40 MPa/sec und in einer Phase P4b der Druck von 20 MPa auf 0 MPa Überdruck mit einem Druckabbau von ca. 10 MPa/sec beaufschlagt wird.

Nach dem Verpressen erfolgt ein mechanisches Bearbeiten durch Fräsen, wobei zunächst eine Grobbearbeitung und sodann eine Feinbearbeitung durchgeführt werden kann. Das Bearbeiten kann ohne Kühlung erfolgen. Eine trockene Bearbeitung ist möglich.

Bei der Grobbearbeitung sollten nachstehende Fräsparameter beachtet werden:

| | |
|---|---|
| Fräserdurchmesser: | 2 bis 5 mm, besonders 2 bis 3 mm |
| Vorschub: | 500 bis 4000 mm/min, besonders 2000 bis 3000 mm/min |
| Seitliche Zustellung ae: | 0,2 bis 3 mm, besonders 1 mm bis 2 mm |
| Tiefenzustellung ap: | 0,1 bis 2 mm, besonders 0,5 mm bis 1 mm |
| Fräserdrehzahl: | 10.000 bis 50.000 1/min, besonders 10.000 bis 20.000 1/min. |

Das Fräswerkzeug sollte insbesondere ein Hartmetallfräser sein.

Fräsparameter für die Feinbearbeitung sollten sein:

| | |
|---|---|
| Fräserdurchmesser: | 0,3 bis 1,5 mm, besonders 0,5 bis 1,0 mm |
| Vorschub: | 300 bis 2000 mm/min, besonders 800 bis 1500 mm/min |
| Seitliche Zustellung ae: | 0,2 bis 0,6 mm, besonders 0,1 mm bis 0,2 mm |
| Tiefenzustellung ap: | 0,05 bis 0,3 mm, besonders 0,1 mm bis 0,15 mm |
| Fräserdrehzahl: | 20.000 bis 60.000 1/min, besonders 25.000 bis 35.000 1/min. |

Das Fräswerkzeug sollte insbesondere ein Hartmetallfräser sein.

Bevorzugterweise wird ein Kugelradiusfräser aus Hartmetall, der mit Titannitrid beschichtet sein kann, verwendet. Hierbei sind nachstehende Schneidenwinkel zu bevorzugen:

| | |
|---|---|
| Spanwinkel: | 0° bis -13°, besonders -9° bis -11° |
| Freiwinkel: | 0° bis 15°, besonders 11° bis 13° |
| Keilwinkel: | ergibt sich 90° minus Freiwinkel minus Spanwinkel . |

Aufgrund der Dichte des Rohlings und des kristallinen Anteils können dentale Formteile mit filigranen Rändern problemlos hergestellt werden. Bei Kronen hat sich gezeigt, dass sich stabile auslaufende Randdicken zwischen 0,05 mm und 0,4 mm ergeben.

Nach dem Herausarbeiten ist das aus dem Rohling hergestellte Formteil als Vorformteil zu bezeichnen, da dieses gegenüber dem dichtgesinterten dentalen Formteil entsprechend dem Schwund des Rohlingmaterials ein Übermaß aufweist. In Abhängigkeit von der Dichte des Rohlings wird das Übermaß berechnet, um einen hochpräzisen Zahnersatz als Endprodukt nach dem Dichtsintern zur Verfügung zu stellen.

Das Durch- bzw. Dichtsintern erfolgt bei einer Temperatur T₂ zwischen 800 °C und 1050°C über eine Haltezeit t₂ zwischen 5 min und 60 min. Haltezeit bedeutet dabei, dass der Rohling bei der gewünschten Endsintertemperatur auf dieser Temperatur gehalten wird.

Beim Durchsintern wird das Vorformteil auf eine feuerfeste Unterlage wie Brennwatte oder auf zunderfreie Metallschichten angeordnet. Stützstrukturen sind nicht erforderlich, da aufgrund der vorhergehenden Kristallisation des Ausgangspulvers die Formstabilität gesichert wird.

## Patentansprüche

1. Verfahren zur Herstellung eines dentalen Formteils wie Inlay, Onlay, Krone oder Brücke, umfassend die Verfahrensschritte:
- Herstellen einer Schmelze der Zusammensetzung in Gew.-%:
| | |
|---|---|
| SiO2 | 46,0 - 72,0 |
| Li2O | 10,0 - 25,0 |
| Zr02 | 6,5 - 14,0 |
| P2O5 | 1,0 - 10,0 |
| Al2O3 | 0,1 - 8,0 |
| K2O | 0,1 - 5,0 |
| CeO2 | 0,1 - 4,0 |
| B2O3 | 0,0 - 4,0 |
| Na2O | 0,0 - 4,0 |
| Tb407 | 0,0 - 2,5 |
sowie 0,0 bis 4,0 von zumindest einem Additiv,
- Herstellen einer Glasfritte durch Verdüsen der Schmelze und Abschrecken in einem Medium,
- gegebenenfalls Erzeugen von Glaspulverpartikeln aus der Glasfritte mit einer Korngrößenverteilung d₉₀ ≤ 80 µm,
- Auskristallisieren von Lithiumsilikatkristallen mit einem Volumenanteil zwischen 10 % und 90 % durch eine erste Wärmebehandlung von entweder der Glasfritte oder den Glaspulverpartikeln in einem ersten Temperaturbereich bei einer Temperatur T₁ mit 500 °C ≤ T₁ ≤ 750 °C über eine Zeit t₁ mit 5 min ≤ t₁ ≤ 120 min,
- wobei dann, wenn die Glasfritte einer Wärmebehandlung unterzogen wird, aus der wärmebehandelten Glasfritte Herstellen von Glaskeramikpartikeln mit einer Korngrößenverteilung d₉₀ ≤ 80 µm erfolgt,
- Pressen der Glaskeramikpulverpartikel zu einem Rohling mit einer Dichte zwischen 30 % und 60 % der theoretischen Dichte des Rohlingmaterials von etwa 2,64 g/cm³,
- Bearbeiten des Rohlings durch Fräsen zur Herstellung eines dem dentalen Formteil entsprechenden Vorformteils unter Berücksichtigung des Schwundverhaltens des Rohlings und
- Durchsintern des Vorformteils bei einer Temperatur T₂ mit 800 °C ≤ T₂ ≤ 1050 °C über eine Zeit t₂ mit 5 min ≤ t₂ ≤ 60 min.

2. Verfahren nach Anspruch 1,
**dadurch gekennzeichnet ,**
**dass** eine Schmelze der Zusammensetzung in Gew.-% hergestellt wird:
| | |
|---|---|
| SiO2 | 49,0 - 69,0 |
| Li20 | 11,5 - 24,0 |
| ZrO2 | 7,0 - 13,5 |
| P2O5 | 1,5 - 9,0 |
| Al2O3 | 0,2 - 7,5 |
| K2O | 0,2 - 4,5 |
| CeO2 | 0,2 - 3,5 |
| B2O3 | 0,0 - 3,5 |
| Na2O | 0,0 - 3,5 |
| Tb4O7 | 0,0 - 2,0 |
sowie 0,0 bis 4,0 von zumindest einem Additiv.

3. Verfahren nach Anspruch 1,
**dadurch gekennzeichnet,**
**dass** eine Schmelze der Zusammensetzung in Gew.-% hergestellt wird:
| | |
|---|---|
| SiO2 | 52,0 - 66,0 |
| Li2O | 12,0 - 22,5 |
| Zr02 | 7,5 - 13,0 |
| P2O5 | 2,0 - 8,5 |
| Al2O3 | 0,3 - 7,0 |
| K2O | 0,3 - 4,0 |
| CeO2 | 0,3 - 3,5 |
| B2O3 | 0,0 - 3,0 |
| Na2O | 0,0 - 3,0 |
| Tb4O7 | 0,0 - 2,0 |
sowie 0,0 bis 4,0 von zumindest einem Additiv.

4. Verfahren nach Anspruch 1,
**dadurch gekennzeichnet ,**
**dass** eine Schmelze der Zusammensetzung in Gew.-% hergestellt wird:
| | |
|---|---|
| SiO2 | 55,0 - 63,0 |
| Li2O | 12,5 - 21,5 |
| ZrO2 | 8,0 - 12,0 |
| P2O5 | 2,5 - 8,0 |
| Al2O3 | 0,4 - 6,5 |
| K2O | 0,4 - 4,0 |
| CeO2 | 0,5 - 3,0 |
| B2O3 | 0,0 - 3,0 |
| Na2O | 0,0 - 3,0 |
| Tb4O7 | 0,0 - 2,0 |
sowie 0,0 bis 4,0 von zumindest einem Additiv.

5. Verfahren nach Anspruch 1,
**dadurch gekennzeichnet ,**
**dass** eine Schmelze der Zusammensetzung in Gew.-% hergestellt wird:
| | |
|---|---|
| SiO2 | 58 - 60 |
| Li2O | 13,5 - 20,5 |
| Zr02 | 8,5 - 11,5 |
| P2O5 | 3,0 - 7,5 |
| Al2O3 | 0,5 - 6,0 |
| K2O | 0,5 - 3,5 |
| CeO2 | 0,5 - 2,5 |
| B2O3 | 0 - 3 |
| Na2O | 0 - 3 |
| Tb407 | 0 - 1,5 |
sowie 0,0 bis 4,0 von zumindest einem Additiv.

6. Verfahren nach zumindest einem der Ansprüche 1 bis 5,
**dadurch gekennzeichnet,**
**dass** der Rohling vor der Bearbeitung und nach der ersten Wärmebehandlung bei einer Temperatur T₃ mit 750 °C ≤ T₃ ≤ 900 °C über eine Zeit t₃ mit 5 min ≤ t₃ ≤ 30 min getempert wird.

7. Verfahren nach zumindest einem der Ansprüche 1 bis 6,
**dadurch gekennzeichnet ,**
**dass** zur Herstellung eines Rohlings in Scheibengeometrie die Glaskeramikpulverpartikel zunächst axial verpresst und sodann nach Einbringen in eine Umhüllende wie einem Beutel aus innenseitig beschichtetem Polyethylen isostatisch nachverdichtet werden, wobei das Nachverdichten insbesondere bei einem Druck pₙ mit 250 MPa ≤ pₙ ≤ 350 MPa über eine Zeit t₄ mit 5 sec ≤ t₄ ≤ 30 sec, insbesondere 5 sec ≤ t₄ ≤ 15 sec durchgeführt wird.

8. Verfahren nach zumindest einem der Ansprüche 1 bis 6,
**dadurch gekennzeichnet ,**
**dass** zur Herstellung eines Rohlings in Quadergeometrie die Glaskeramikpulverpartikel sukzessiv insbesondere kontinuierlich mit ansteigendem Druck über eine Zeit t₅ axial verpresst werden, wobei maximaler Druck p₅ beträgt 50 MPa ≤ p₅ ≤ 400MPa, insbesondere 100 MPa ≤ p₅ ≤ 200 MPa.

9. Verfahren nach zumindest einem der Ansprüche 1 bis 6,
**dadurch gekennzeichnet,**
**dass** zur Herstellung eines stabförmigen Rohlings, insbesondere zylindrischer Geometrie, das Glaskeramikpulver in eine schlauchförmige Pressform, insbesondere aus Polyurethan eingebracht und sodann quasi isostatisch verpresst wird.

10. Verfahren nach zumindest einem der Ansprüche 1 bis 9,
**dadurch gekennzeichnet,**
**dass** der Rohling durch Fräsen zumindest grob bearbeitet und sodann feinbearbeitet wird, wobei zur Grobbearbeitung bevorzugte Fräsparameter berücksichtigt werden:
| | |
|---|---|
| Fräserdurchmesser: | 2 bis 5 mm, besonders 2 bis 3 mm |
| Vorschub: | 500 bis 4000 mm/min, besonders 2000 bis 3000 mm/min |
| Seitliche Zustellung ae: | 0,2 bis 3 mm, besonders 1 mm bis 2 mm |
| Tiefenzustellung ap: | 0,1 bis 2 mm, besonders 0,5 mm bis 1 mm |
| Fräserdrehzahl: | 10.000 bis 50.000 1/min, besonders 10.000 bis 20.000 1/min. |
und/oder zur Feinbearbeitung bevorzugte Fräsparameter berücksichtigt werden:
| | |
|---|---|
| Fräserdurchmesser: | 0,3 bis 1,5 mm, besonders 0,5 bis 1,0 mm |
| Vorschub: | 300 bis 2000 mm/min, besonders 800 bis 1500 mm/min |
| Seitliche Zustellung ae: | 0,2 bis 0,6 mm, besonders 0,1 mm bis 0,2 mm |
| Tiefenzustellung ap: | 0,05 bis 0,3 mm, besonders 0,1 mm bis 0,15 mm |
| Fräserdrehzahl: | 20.000 bis 60.000 1/min, besonders 25.000 bis 35.000 1/min. |

11. Verfahren nach zumindest einem der Ansprüche 1 bis 10,
**dadurch gekennzeichnet ,**
**dass** als Fräser ein Kugelradiusfräser mit folgenden Schneidenwinkeln verwendet wird:
| | |
|---|---|
| Spanwinkel: | 0° bis -13°, besonders -9° bis -11° |
| Freiwinkel: | 0° bis 15°, besonders 11° bis 13° |
| Keilwinkel: | ergibt sich 90° minus Freiwinkel minus Spanwinkel |

12. Verfahren nach zumindest Anspruch 1,
**dadurch gekennzeichnet,**
**dass** der Rohling in Kieselsäure oder Alkalisilikatlösung (Wasserglas) getaucht, getrocknet und sodann durch Fräsen trocken bearbeitet wird oder dass der Rohling durch Fräsen bearbeitet und sodann, vor der Sinterung auf Enddichte, in Kieselsäure oder Alkalisilikatlösung (Wasserglas) getaucht und getrocknet wird.

## Claims

1. A method for producing a dental molded part such as an inlay, onlay, crown, or bridge, comprising the method steps of:
- Producing a molten mass with a composition (in % by weight):
| | |
|---|---|
| SiO2 | 46.0-72.0 |
| Li2O | 10.0-25.0 |
| ZrO2 | 6.5-14.0 |
| P2O5 | 1.0-10.0 |
| Al2O3 | 0.1-8.0 |
| K2O | 0.1-5.0 |
| CeO2 | 0.1-4.0 |
| B2O3 | 0.0-4.0 |
| Na2O | 0.0-4.0 |
| Tb4O7 | 0.0-2.5 |
as well as 0.0 to 4.0 of at least one additive,
- Producing a glass frit by atomizing the molten mass and quenching in a medium,
- If applicable, generating glass powder particles from the glass frit with a grain size distribution d₉₀ < 80 µm,
- Final crystallization of lithium silicate crystals with a volume fraction of between 10 % and 90 % by a first thermal treatment from either the glass frit or the glass powder particles in a first temperature range at a temperature T₁ with 500 °C ≤ T₁ ≤ 750 °C for a duration t₁ with 5 min ≤ t₁ ≤ 120 min,
- Whereby at a time when the glass frit has been subjected to a thermal treatment, one produces glass-ceramic particles with a grain size distribution d₉₀ ≤ 80 µm from the heat-treated glass frit,
- Pressing the glass-ceramic powder particles to form a blank with a density between 30 % and 60 % of the theoretical density of the material of the blank of around 2,6 g/cm³,
- Machining the blank by milling to produce a pre-form part that corresponds to the dental molded part under consideration of the shrinkage characteristics of the blank, and
- Sintering the preformed part to completion at a temperature T₂ with 800 °C ≤ T₂ ≤ 1050 °C for a time period t₂ with 5 min ≤ t₂ ≤ 60 min.

2. The method of claim 1,
**characterized in that**
one produces a molten mass with a composition (in % by weight):
| | |
|---|---|
| SiO2 | 49.0-69.0 |
| Li2O | 11.5-24.0 |
| ZrO2 | 7.0-13.5 |
| P2O5 | 1.5-9.0 |
| Al2O3 | 0.2-7.5 |
| K2O | 0.2-4.5 |
| CeO2 | 0.2-3.5 |
| B2O3 | 0.0-3.5 |
| Na2O | 0.0-3.5 |
| Tb4O7 | 0.0-2.0 |
as well as 0.0 to 4.0 of at least one additive.

3. The method of claim 1,
**characterized in that**
one produces a molten mass with the composition (in % by weight):
| | |
|---|---|
| SiO2 | 52.0-66.0 |
| Li2O | 12.0-22.5 |
| ZrO2 | 7.5-13.0 |
| P2O5 | 2.0-8.5 |
| Al2O3 | 0.3-7.0 |
| K2O | 0.3-4.0 |
| CeO2 | 0.3-3.5 |
| B2O3 | 0.0-3.0 |
| Na2O | 0.0-3.0 |
| Tb4O7 | 0.0-2.0 |
as well as 0.0 to 4.0 of at least one additive.

4. The method of claim 1,
**characterized in that**
one produces a molten mass with a composition (in % by weight):
| | |
|---|---|
| SiO2 | 55.0-63.0 |
| Li2O | 12.5-21.5 |
| ZrO2 | 8.0-12.0 |
| P2O5 | 2.5-8.0 |
| Al2O3 | 0.4-6.5 |
| K2O | 0.4-4.0 |
| CeO2 | 0.5-3.0 |
| B2O3 | 0.0-3.0 |
| Na20 | 0.0-3.0 |
| Tb4O7 | 0.0-2.0 |
as well as 0.0 to 4.0 of at least one additive.

5. The method of claim 1,
**characterized in that**
one produces a molten mass with a composition (in % by weight):
| | |
|---|---|
| SiO2 | 58-60 |
| Li2O | 13.5-20.5 |
| ZrO2 | 8.5-11.5 |
| P2O5 | 3.0-7.5 |
| Al2O3 | 0.5-6.0 |
| K2O | 0.5-3.5 |
| CeO2 | 0.5-2.5 |
| B2O3 | 0-3 |
| Na2O | 0-3 |
| Tb4O7 | 0-1.5 |
as well as 0.0 to 4.0 of at least one additive.

6. The method of at least one of claims 1 to 5,
**characterized in that** prior to machining and after the first thermal treatment, the blank is tempered at a temperature T₃ with 750 °C ≤ T₃ ≤ 900 °C for a duration t₃ with 5 min ≤ t₃ ≤ 30 min.

7. The method of at least one of claims 1 to 6,
**characterized in that**
to produce a blank with a disk geometry, the glass-ceramic powder particles are at first axial pressed and subsequently, upon insertion into an encasing element such as a pouch coated with polyethylene on its inside, are subjected to isostatic re-compression, whereby the re-compression in particular is performed at a pressure pₙ with 250 MPa ≤ pₙ ≤ 350 MPa for a time t₄ with 5 sec ≤ t4 ≤ 30 sec, in particular 5 sec ≤ t₄ ≤ 15 sec.

8. The method of at least one of claims I to 6,
**characterized in that**
for the production of a blank with a cube geometry, the glass-ceramic powder particles are axially pressed successively and in particular continuously with increasing pressure for a duration t₅, whereby the maximum pressure is p₅, with 50 MPa ≤ p₅ ≤ 400MPa, in particular 100 MPa ≤ p₅ ≤ 200 MPa.

9. The method of at least one of claims 1 to 6,
**characterized in that**
for the production of a rod-shaped blank, in particular with cylindrical geometry, the glass-ceramic powder is introduced into a tubular press mould, in particular of polyurethane and subsequently is subjected to quasi-isostatic pressing.

10. The method of at least one of claims 1 to 9,
**characterized in that**
the blank is subjected to at least a first coarse machining by milling and subsequent precision machining, whereby preferred milling parameters for the coarse machining should be taken into account:
| | |
|---|---|
| Cutter diameter: | 2 to 5 mm, in particular 2 to 3 mm |
| Feed: | 500 to 4000 mm/min, in particular 2000 to 3000 mm/min |
| Lateral feed ae: | 0.2 to 3 mm, in particular 1 mm to 2 mm |
| Depth feed ap: | 0.1 to 2 mm, in particular 0.5 mm to 1 mm |
| Cutter speed: | 10.000 to 50.0001/min, in particular 10.000 to 20.000 1/min. |
and/or one takes into account preferred milling parameters for the precision machining:
| | |
|---|---|
| Cutter diameter: | 0.3 to 1.5 mm, in particular 0.5 to 1.0 mm |
| Feed: | 300 to 2000 mm/min, in particular 800 to 1500 mm/min |
| Lateral feed ae: | 0.2 to 0.6 mm, in particular 0.1 mm to 0.2 mm |
| Depth feed ap: | 0.05 to 0.3 mm, in particular 0.1 mm to 0.15 mm |
| Cutter speed: | 20,000 to 60,000 1/min, in particular 25,000 to 35,000 1/min. |

11. The method of at least one of claims 1 to 10,
**characterized in that**
the employed cutter is a radius cutter with the following cutting edge angles:
| | |
|---|---|
| Cutting angle: | 0° to -13°, in particular -9° to -11° |
| Clearance angle: | 0° to 15°, in particular 11° to 13° |
| Wedge angle: | Results from: 90° minus clearance angle minus cutting angle. |

12. The method of at least claim 1,
**characterized in that**
the blank is immersed in silicic acid or in an alkali silicate solution (soluble glass), is dried, and subsequently is machined by dry milling, or **in that** the blank is machined by milling and subsequently, prior to the sintering to the final density, is immersed in silicic acid or alkali silicate solution (soluble glass) and subsequently dried.

## Revendications

1. Procédé pour la production d'une pièce façonnée dentaire, telle qu'un inlay, un onlay, une couronne ou un bridge, comprenant les étapes de procédé de :
- préparation d'une masse fondue de la composition présentant, en % en poids :
| | |
|---|---|
| SiO2 | 46,0 - 72,0 |
| Li2O | 10,0 - 25,0 |
| ZrO2 | 6,5 - 14,0 |
| P2O5 | 1,0 - 10,0 |
| Al2O3 | 0,1 - 8,0 |
| K2O | 0,1 - 5,0 |
| CeO2 | 0,1 - 4,0 |
| B2O3 | 0,0 - 4,0 |
| Na2O | 0,0 - 4,0 |
| Tb4O7 | 0,0 - 2,5 |
ainsi que 0,0 à 4,0 d'au moins un additif,
- préparation d'une fritte de verre par pulvérisation de la masse fondue et refroidissement brusque dans un milieu,
- le cas échéant génération de particules de poudre de verre, présentant une distribution granulométrique d₉₀ ≤ 80 µm, à partir de la fritte de verre,
- cristallisation de cristaux de silicate de lithium présentant une proportion volumique entre 10 % et 90 % par un premier traitement thermique, soit de la fritte de verre, soit des particules de poudre de verre dans une première plage de température T₁ où 500 °C ≤ T₁ ≤ 750 °C sur une période t₁ où 5 min ≤ t₁ ≤ 120 min,
- où, si c'est la fritte de verre qui est soumise à un traitement thermique, une préparation de particules de vitrocéramique présentant une distribution granulométrique d₉₀ ≤ 80 µm a lieu ensuite,
- pressage des particules de poudre de vitrocéramique en une ébauche présentant une densité entre 30 % et 60 % de la densité théorique de l'ébauche d'environ 2,64 g/cm³,
- usinage de l'ébauche par fraisage pour la production d'une pièce préfaçonnée correspondant à la pièce façonnée dentaire, tenant compte du comportement de retrait de l'ébauche.
- frittage global de la pièce préfaçonnée à une température T₂ où 800 °C ≤ T₂ ≤ 1050 °C sur une période t₂ où 5 min ≤ t₂ ≤ 60 min.

2. Procédé selon la revendication 1,
**caractérisé en ce**
**qu'**une masse fondue de la composition suivante, en % en poids, est préparée :
| | |
|---|---|
| SiO2 | 49,0 - 69,0 |
| Li2O | 11,5 - 24,0 |
| Zr02 | 7,0 - 13,5 |
| P2O5 | 1,5 - 9,0 |
| Al2O3 | 0,2 - 7,5 |
| K2O | 0,2 - 4,5 |
| CeO2 | 0,2 - 3,5 |
| B2O3 | 0,0 - 3,5 |
| Na2O | 0,0 - 3,5 |
| Tb4O7 | 0,0 - 2,0 |
ainsi que 0,0 à 4,0 d'au moins un additif.

3. Procédé selon la revendication 1,
**caractérisé en ce**
**qu'**une masse fondue de la composition suivante, en % en poids, est préparée :
| | |
|---|---|
| SiO2 | 52,0 - 66,0 |
| Li2O | 12,0 - 22,5 |
| ZrO2 | 7,5 - 13,0 |
| P2O5 | 2,0 - 8,5 |
| Al2O3 | 0,3 - 7,0 |
| K2O | 0,3 - 4,0 |
| CeO2 | 0,3 - 3,5 |
| B2O3 | 0,0 - 3,0 |
| Na2O | 0,0 - 3,0 |
| Tb4O7 | 0,0 - 2,0 |
ainsi que 0,0 à 4,0 d'au moins un additif.

4. Procédé selon la revendication 1,
**caractérisé en ce**
**qu'**une masse fondue de la composition suivante, en % en poids est préparée :
| | |
|---|---|
| Si02 | 55,0 - 63,0 |
| Li2O | 12,5 - 21,5 |
| ZrO2 | 8,0 - 12,0 |
| P2O5 | 2,5 - 8,0 |
| Al2O3 | 0,4 - 6,5 |
| K2O | 0,4 - 4,0 |
| CeO2 | 0,5 - 3,0 |
| B2O3 | 0,0 - 3,0 |
| Na2O | 0,0 - 3,0 |
| Tb4O7 | 0,0 - 2,0 |
ainsi que 0,0 à 4,0 d'au moins un additif.

5. Procédé selon la revendication 1,
**caractérisé en ce**
**qu'**une masse fondue de la composition suivante, en % en poids, est préparée :
| | |
|---|---|
| SiO2 | 58 - 60 |
| Li20 | 13,5 - 20,5 |
| ZrO2 | 8,5- 11,5 |
| P2O5 | 3,0 - 7,5 |
| Al2O3 | 0,5 - 6,0 |
| K2O | 0,5 - 3,5 |
| Ce02 | 0,5 - 2,5 |
| B2O3 | 0 - 3 |
| Na2O | 0 - 3 |
| Tb4O7 | 0 - 1,5 |
ainsi que 0,0 à 4,0 d'au moins un additif.

6. Procédé selon au moins l'une quelconque des revendications 1 à 5,
**caractérisé en ce**
**que** l'ébauche est recuite avant l'usinage et après le premier traitement thermique à une température T₃ où 750 °C ≤ T₃ ≤ 900 °C sur une période t₃ où 5 min ≤ t₃ ≤ 30 min.

7. Procédé selon au moins l'une quelconque des revendications 1 à 6,
**caractérisé en ce**
**que**, pour la production d'une ébauche pourvue d'une géométrie d'une plaque, les particules de poudre de vitrocéramique sont d'abord pressées axialement et ensuite post-compactées de manière isostatique après l'introduction dans une enveloppe telle qu'une poche en polyéthylène revêtu côté interne, le post-compactage étant réalisé à une pression pₙ où 250 MPa ≤ pₙ ≤ 350 MPa sur une période t₄ où 5 sec ≤ t₄ ≤ 30 sec, en particulier 5 sec ≤ t₄ ≤ 15 sec.

8. Procédé selon au moins l'une quelconque des revendications 1 à 6,
**caractérisé en ce**
**que**, pour la production d'une ébauche pourvue d'une géométrie parallélépipédique, les particules de poudre de vitrocéramique sont pressées axialement successivement, en particulier en continu, à une pression croissante sur une période ts, la pression maximale p₅ étant 50 MPa ≤ p₅ ≤ 400 MPa, en particulier 100 MPa ≤ ps ≤ 200 MPa.

9. Procédé selon au moins l'une quelconque des revendications 1 à 6,
**caractérisé en ce**
**que**, pour la production d'une ébauche en forme de barre, pourvue en particulier d'une géométrie cylindrique, la poudre de vitrocéramique est introduite dans un moule de pressage en forme de tuyau, en particulier en polyuréthane et ensuite pressée de manière quasi-isostatique.

10. Procédé selon au moins l'une quelconque des revendications 1 à 9,
**caractérisé en ce**
**que** l'ébauche est usinée par fraisage au moins grossièrement et ensuite soumise à un usinage fin, les paramètres de fraisage préférés suivants étant pris en considération pour l'usinage grossier :
| | |
|---|---|
| diamètre de la fraise : | 2 à 5 mm, en particulier 2 à 3 mm |
| | |
| avancement : | 500 à 4000 mm/min, en particulier 2000 à 3000 mm/min |
| passe latérale ae : | 0,2 à 3 mm, en particulier 1 à 2 mm |
| profondeur de passe ap : | 0,1 à 2 mm, en particulier 0,5 à 1 mm |
| | |
| vitesse de rotation de la fraise : | 10 000 à 50 000 1/min, en particulier 10 000 à 20 000 1/min, |
et/ou les paramètres de fraisage préférés suivants étant pris en considération pour l'usinage fin :
| | |
|---|---|
| diamètre de la fraise : | 0,3 à 1,5 mm, en particulier 0,5 à 1,0 mm |
| avancement : | 300 à 2000 mm/min, en particulier 800 à 1500 mm/min |
| passe latérale ae : | 0,2 à 0,6 mm, en particulier 1 à 0,2 mm |
| profondeur de passe ap : | 0,05 à 0,3 mm, en particulier 1 à 0,15 mm |
| vitesse de rotation de la fraise : | 20 000 à 60 000 1/min, en particulier 10 000 à 35 000 1/min. |

11. Procédé selon au moins l'une quelconque des revendications 1 à 10,
**caractérisé en ce**
**qu'**une fraise à rayon sphérique, présentant les angles de coupe suivants, est utilisée comme fraise :
| | |
|---|---|
| angle de coupe : | 0° à -13°, en particulier -9° à -11 ° |
| angle d'attaque : | 0° à 15°, en particulier 11° à 13° |
| angle de coin: | est obtenu par 90° moins l'angle d'attaque moins l'angle de coupe. |

12. Procédé selon au moins la revendication 1,
**caractérisé en ce**
**que** l'ébauche est immergée dans de la silice ou une solution de silicate de métal alcalin (verre soluble), séchée et ensuite usinée à sec par fraisage ou en ce que l'ébauche est usinée par fraisage et ensuite immergée dans de la silice ou une solution de silicate de métal alcalin (verre soluble), avant le frittage à la densité finale.
